# EUROPEAN PATENT APPLICATION

(11) **EP 3 901 677 A1**
(43) Date of publication of application: **27.10.2021**
(21) Application number: 20170632.2
(22) Date of filing: 21.04.2020
(51) Int. Cl.: G02B 6/42, A61B 18/24, H05K 1/18

(54) **OPTICAL TRANSMISSION OF SIGNALS TO OR FROM AN ELECTRONIC ELEMENT**

(71) Applicant: Koninklijke Philips N.V., 5656 AG Eindhoven (NL); Technische Universiteit Delft, 2628 CN Delft (NL)
(72) Inventor: DEKKER, Ronald, 5656 AE Eindhoven (NL); LI, Jian, 5656 AE Eindhoven (NL); HENNEKEN, Vincent Adrianus, 5656 AE Eindhoven (NL)
(74) Representative: Philips Intellectual Property & Standards

(57) **Abstract**

An interconnection unit is for coupling an optical unit (an optical transmitter or an optical receiver) to an optical fiber. A contact layer has a first portion for connection to the optical unit and a second portion for electrical and mechanical connection of the interconnection unit to a carrier (for carrying control circuitry). The contact layer is configurable with an angle between the first and second portions. A receiving block which carries the first portion of the contact layer provides alignment of the end of an optical fiber with a light emitting region or light receiving region of the optical unit.

## Description

### FIELD OF THE INVENTION

The invention relates to the optical transmission of electronic element signals in a small size application. Particularly, but not exclusively, it is of interest for the transmission of sensor signals along a catheter. For example, it relates to an interconnection unit for coupling the output of a sensor element to an optical fiber via an integrated circuit laser or an interconnection unit for coupling the optical fiber to a receiving element such as a photodiode.

### BACKGROUND OF THE INVENTION

Sensing catheters are well known. There are various types of sensor that may be mounted on a catheter, including low data volume sensors such as temperature sensors and pressure sensors but also high data volume sensors such as imaging sensors. It is for example known to provide a catheter with ultrasound imaging capability.

In sensing catheters producing high data rates, such as ultrasound imaging catheters, it may be challenging to transport the high data rates from the catheter to the acquisition system outside of the patient's body. Conventionally, electrical wires are used for this data transport, which are often thin and consequently have low electrical performance. At increasing data rates, e.g. needed for digitization at the catheter tip, it may become necessary to move to optical data transmission, as the bandwidth of the thin wires is no longer sufficient for reliable electrical signal transmission.

The most suitable light sources for high-speed optical data transmission from inside a catheter are vertical cavity surface emitting lasers, VCSELs, because they combine relatively high data rate transmission capabilities with low power usage. In addition, alignment requirements for coupling a multimode fiber to a VCSEL are quite relaxed. The alternative side emitting lasers exhibit much higher power consumption, and require alignment with much higher accuracy for successful fiber coupling.

Extremely small VCSELs are commercially available, which facilitates the use of this type of lasers in catheter applications.

Despite the usefulness of VCSELs for data transmission from inside a catheter, one main drawback remains. A VCSEL emits light from its top surface, which then should be captured by an optical fiber pointing towards it. Due to their limited minimum bending radius, optical fibers need to be positioned along the longitudinal direction of the catheter. Consequently, the light emitting surface of the VCSEL should be placed perpendicularly to the catheter length direction, unless the light path is redirected from out-of-plane to along the plane of the VCSEL surface by some additional optical component, such as a mirror. Due to the severe size restrictions inside a catheter, often too little space is available to add such optical components. Moreover, aligning these optical components as well as the fiber to the VCSEL's emitting surface adds to the assembly cost.

The alternative of mounting the VCSEL perpendicularly to the catheter length direction is problematic, because the substrate for mounting the electrical components needed for the sensor functionality is generally oriented along the catheter length direction, as this is normally the direction in which most space is available. Having the VCSEL in an upright position on such a longitudinally oriented substrate including the necessary electrical connections is difficult, as the VCSEL's contact pads are located on its top and/or bottom side.

The reception of the optical signal requires an optical receiver such as a photodiode coupled to the optical fiber.

There is a need for an improved arrangement for providing optical sensor signals along a catheter or more generally for a coupling to an optical fiber (at the transmitter end and/or the receiver end), which can be used when there are space constraints.

### SUMMARY OF THE INVENTION

The invention is defined by the claims.

According to examples in accordance with an aspect of the invention, there is provided an interconnection unit for coupling an optical unit, which comprises an optical transmitter having an optical emitting region or an optical receiver having an optical receiving region and an attachment surface, to an optical fiber, comprising:
a contact layer having:
   a first portion for connection to the attachment surface of the optical unit; and
   a second portion for electrical and mechanical connection of the interconnection unit to a carrier, wherein the contact layer is configurable with an about 90 degree angle between the first and second portions; and
a receiving block for receiving the optical fiber, wherein the first portion of the contact layer is over an end face of the receiving block, and the receiving block is for aligning the end of the optical fiber with the optical emitting region or optical receiving region of the optical unit.

This interconnection unit enables an about 90 degree angle to be formed between the face that connects to the optical unit attachment surface (e.g. the light emitting surface of a vertical cavity surface emitting laser) and the face that connects electrically and mechanically to a carrier (e.g. a flexible printed circuit board, which may then carry other circuit components).

In one set of examples, the optical unit comprises an integrated circuit laser, wherein the attachment surface includes the optical emitting region and electrical connection regions. In this case, the laser has electrical connections and the optical emitting region on one side. The first portion of the contact layer connects to this attachment surface, and the 90 degree angle is for example implemented by a single 90 degree bend to the second portion of the contact layer, which connects to the carrier.

In another set of examples, the optical unit comprises an integrated circuit laser, wherein the attachment surface includes electrical connection regions and is opposite a surface which includes the optical emitting region. In this case, the laser has electrical connections on one side and the optical emitting region on an opposite side. The first portion of the contact layer connects to the attachment surface, but the 90 degree angle is for example implemented by three 90 degree bends (so that he optical emitting region faces towards the optical fiber and the attachment surface faces away from the optical fiber).

In another set of examples, the optical unit comprises a photodiode, wherein the attachment surface includes electrical connection regions and is opposite a surface which includes the optical receiving region. In this case, a photodiode has electrical connections on one side and the optical receiving region on an opposite side. The first portion of the contact layer connects to the attachment surface, but the 90 degree angle is again implemented by three 90 degree bends (so that he optical receiving region faces towards the optical fiber and the attachment surface faces away from the optical fiber).

When the optical unit is a laser, it is for example for interfacing between another electronic element such as a sensor and the optical fiber. The sensor is for example for positioning along a medical interventional device, e.g. catheter or at its distal end. The first portion can be aligned perpendicularly to an elongate axis of the catheter at the location of the sensor, i.e. facing forwards along the catheter. The second portion can then be aligned parallel to an elongate axis of the catheter at the location of the sensor. Thus, the carrier, e.g. a flexible PCB, runs along the length of the catheter, whereas the attachment surface extends across the catheter. This provides the optimum use of the limited space within the catheter. It enables high speed signal communication over an optical fiber for an electronic element which generates large volumes of data, such as an ultrasound imaging sensor.

In one set of examples, the invention enables a miniature in-plane laser surface-to-fiber connection module to be formed, suitable for optical data transport from the tip of a catheter to the proximal system side. The invention can however be applied to other miniature electronic elements and is not limited to use with catheters. The invention can also be used at the receiving side, where an optical signal carried by the optical fiber is coupled to a receiving optical unit such as a photodiode.

The contact layer for example comprises flexible electrical connections formed over a substrate. This substrate may be comprise thick portions, e.g. to define the receiving block, and thin portions, e.g. to define a bending zone. It may be manufactured in a planar configuration and then bent into its desired end shape at the bending zone as part of an assembly process.

The receiving block for example comprises a through hole for receiving the optical fiber, wherein the through hole extends to the end face. The receiving block thus provides the alignment of the optical fiber with the optical unit, which is mounted over the end face.

The first portion of the contact layer for example comprises contact pads for electrical connection to the optical unit. If the contact layer is between the optical unit and the interconnection unit, the first portion of the contact layer may also have an opening for alignment with the emitting or receiving region.

The interconnection unit may further comprise a support block, wherein the second portion of the contact layer is over the support block. The receiving block and the supporting block are preferably silicon, for example comprising islands formed beneath the contact layer. The interconnection unit may thus be formed by known semiconductor processing techniques.

In particular, the interconnection unit may be formed based on silicon processing to form regions of selectable thickness and to form openings. Between the support block and the receiving block, a thinner silicon region may be present, which is the location at which bending takes place. The contact layer is then a surface treatment of the silicon substrate.

The interconnection unit may be for coupling a plurality of optical units to respective optical fibers,
wherein the first portion is for connection to the attachment surface of each of a plurality of optical units,
wherein the second portion is for electrical and mechanical connection of the interconnection unit to a single carrier,
and wherein the receiving block comprises a plurality of through holes each for receiving a respective optical fiber.

Thus, a compact arrangement is provided enabling multiple electronic element outputs to be converted to optical signals and routed along respective optical fibers.

The invention also provides a sensing catheter system comprising:
a catheter having a proximal end and a distal end;
a sensor element positioned along the catheter or at the distal end, the sensor element generating an electrical sensing signal;
an integrated circuit laser for converting the electrical sensing signal to an optical sensing signal, wherein the integrated circuit laser comprises an attachment surface which comprises an emitting region and electrical connection regions, and the attachment surface is perpendicular to an elongate axis of the catheter at the location of the sensor element;
an optical fiber for relaying the optical sensing signal along the catheter, the optical fiber having an end facing the emitting region;
the interconnection unit as defined above between the integrated circuit laser and the optical fiber, wherein the contact layer of the interconnection unit has a 90 degree angle between the first and second portions such that the second portion is aligned parallel to an elongate axis of the catheter at the location of the sensor element;
a carrier connected to the second portion of the contact layer; and
circuitry for controlling the integrated circuit laser, mounted on the carrier.

A catheter with integrated sensor is thus provided. The sensor signal is converted to an optical signal and this is provided to the end of an optical fiber. The end of the optical fiber and the emitting surface of the integrated circuit laser are perpendicular to the catheter length, whereas the carrier for the control circuitry is parallel to the catheter length, i.e. it extends along the catheter length.

The integrated circuit laser is for example a vertical cavity surface emitting laser, VCSEL and the optical fiber is for example a multimode optical fiber. This allows relaxed conditions for the optical fiber alignment.

The sensing system may comprise a plurality of sensor elements and a respective plurality of optical fibers, wherein the first portion of the contact layer is connected to the attachment surface of each of a plurality of integrated circuit lasers, wherein the second portion of the contact layer is connected to a single carrier, and wherein receiving block comprises a plurality of through holes each for receiving the respective optical fiber.

The invention also provides a method of forming a coupling arrangement for coupling an optical unit, which comprises an optical transmitter having an optical emitting region or an optical receiver having an optical receiving region, and an attachment surface, to an optical fiber, the method comprising:
forming an interconnection unit comprising:
   a planar contact layer having a first portion for connection to an attachment surface of the optical unit and a second portion for electrical and mechanical connection to a carrier;
   a receiving block beneath the first portion of the planar contact layer for receiving the optical fiber and aligning the end of the optical fiber with the optical receiving region or the optical emitting region;
   a support block beneath the second portion of the planar contact layer;
mounting the attachment surface of the optical unit over the first portion of the planar contact layer; and
forming an about 90 degree angle between the first and second portions.

The about 90 degree angle means the first portion can be aligned perpendicular to an axis (e.g. a catheter elongate axis) and the second portion can be aligned substantially parallel to the axis.

The method may further comprise:
mounting the coupling arrangement over the end of an optical fiber; and
coupling the second portion of the planar contact layer to a carrier which carries circuitry for controlling the optical unit.

The receiving block and the support block are preferably formed as silicon substrate regions beneath the contact layer.

The receiving block and support block are for example silicon.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a catheter equipped with ultrasound imaging capability;
Figure 2 shows a perspective view of the catheter in the region of the laser;
Figure 3 shows a first perspective view of an optical link module;
Figure 4 shows a second perspective view of the optical link module;
Figure 5 shows a silicon substrate having through opening and also different thickness of silicon on the underside;
Figure 6 shows the underside of the arrangement of Figure 5;
Figure 7 shows the score lines for cutting support tabs and thereby separating the optical link module;
Figure 8 shows the separated optical link module, with the contact layer still planar;
Figure 9 shows the folding of the contact layer around the bending zone to form the final configuration, and the insertion of the optical fiber;
Figure 10 shows one view of an interconnection unit for coupling the output of a plurality of lasers to respective optical fibers;
Figure 11 shows another view of the interconnection unit of Figure 10; and
Figure 12 shows a modification to the design of an interconnection unit for use with a laser or photodiode with opposing attachment and light emitting or receiving surfaces.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an interconnection unit for coupling an optical unit (an optical transmitter or an optical receiver) to an optical fiber. A contact layer has a first portion for connection to the optical unit and a second portion for electrical and mechanical connection of the interconnection unit to a carrier (for carrying control circuitry). The contact layer is configurable with a 90 degree angle between the first and second portions. A receiving block which carries the first portion of the contact layer provides alignment of the end of an optical fiber with a light emitting region or light receiving region of the optical unit.

The optical unit is for example for interfacing between an electronic element and the optical fiber. The electronic element may be any unit which delivers an output suitable for transmission over an optical fiber, such as a high data rate sensor, for example an ultrasound imaging sensor. Alternatively, the optical unit may be for receiving data transmitted along the optical fiber.

The invention will first be explained using an example in which the optical unit is a laser for transmitting signals into the optical fiber, and in which it is for converting signals from the electrical domain to the optical domain.

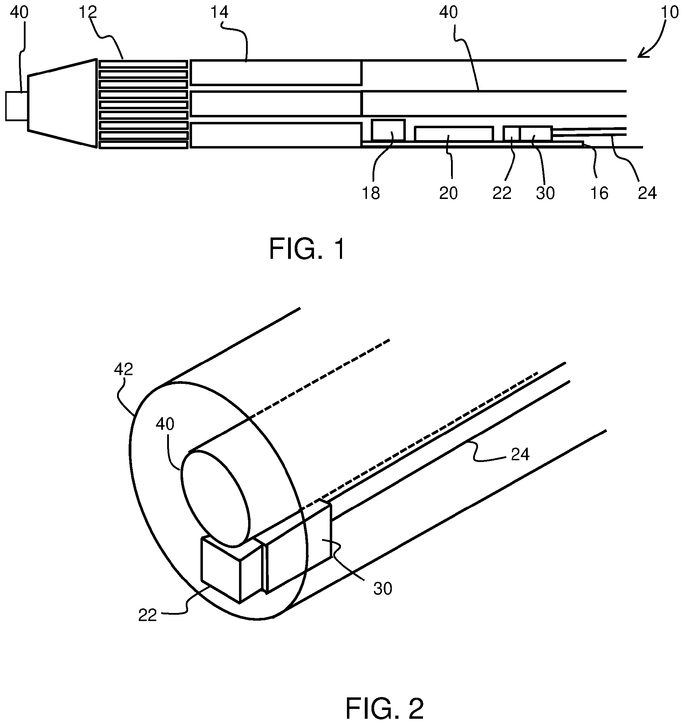

Figure 1 shows an interventional medical device 10, such as an intravascular catheter equipped with ultrasound imaging capability. The catheter has an array of ultrasound transducers 12 at the distal end of the catheter. In the example shown, the ultrasound transducers form an annular ring facing radially outwardly.

The ultrasound transducers are operated by ASIC control circuits 14. Set further back from the distal end is a carrier 16 such as a flexible printed circuit board which carries other circuit components such as capacitors 18, analog to digital converter circuits 20 and an electrical to optical converter in the form of an integrated circuit laser 22. The circuit components may relate to the driving of the laser and/or to the operation of the ultrasound transducers.

The laser converts the ultrasound transducer signals from the electrical domain to the optical domain and couples the optical signal to an optical fiber 24.

The invention in this example provides an interconnection unit 30 coupled to the laser 22, the optical fiber 24 and the carrier 16. It enables the light emitting face of the laser 22, which is also the electrical connection face, to be in a plane perpendicular to the elongate axis of the catheter, whereas the coupling of the interconnection unit 30 to the carrier 16 is in a plane parallel to the elongate axis of the catheter. Thus, the components carried by the carrier can be arranged along the length of the catheter, as shown.

Figure 2 shows a perspective view of the catheter in the region of the laser 22. The catheter has an inner lumen 40 and an outer wall 42. The components are housed in the annular space between them. The inner lumen 40 is for example for deployment over a guidewire. By way of example, the diameter of the inner lumen may be around 0.5 mm and the outer diameter of the catheter may be around 1 mm.

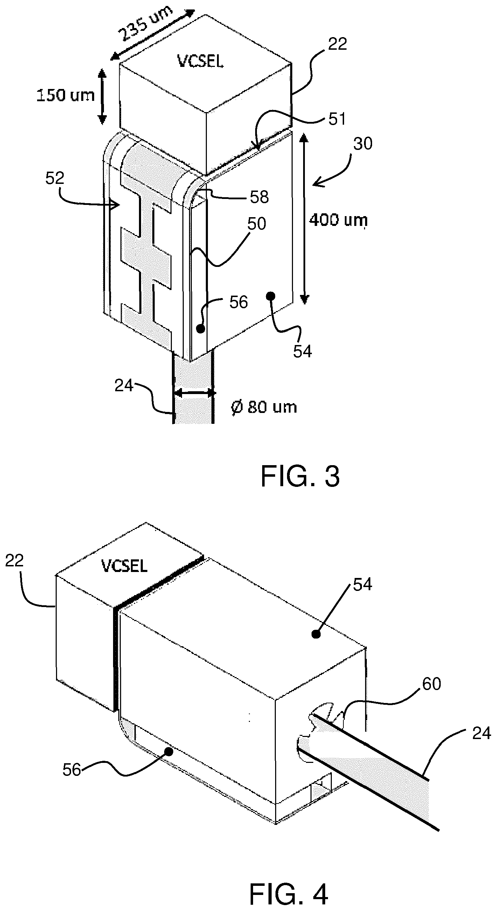

Figure 3 shows a first perspective view of the interconnection unit 30 and the integrated circuit laser 22, such as a vertical cavity surface emitting laser, mounted to the interconnection unit 30. Example dimensions are also shown.

The interconnection unit 30 serves as an interposer between the VCSEL 22 and the optical fiber 30 in order to
(i) facilitate alignment of the optical fiber to the light emitting surface of the VCSEL
(ii) maintain their relative position after fixation and
(iii) route electrical contacts from the VCSEL to the side of the interconnection unit so that the interconnection unit 30 can be mounted sideways to the carrier 16 as described above.

The resulting sub-assembly shown in Figure 3, comprising the interconnection unit 30, VCSEL 22 and fiber end aligned to the light emitting surface of the VCSEL constitutes a miniature in-plane VCSEL-to-fiber connection module. This sub-assembly is referred to as an optical link module.

The optical link module is more generally for use with an integrated circuit laser which has its electrical contacts and laser emitting spot on the same surface, of which a VCSEL is an example. This is surface described as an attachment surface. The interconnection unit dimensions can be adjusted to the laser dimensions.

As an example, one suitable commercially available VCSEL has outer dimensions of 235 µm x 235 µm x 150 µm. This will be used as an example to show the suitable appropriate dimensions for the other components. However, this single example of suitable dimensions is not intended to be limiting and is merely presented to give an understanding of the general scale involved.

For this example, the entire dimension of the optical link module is approximately 240 µm x 280 µm x 600 µm.

The interconnection unit 30 incorporates flexible interconnects to reroute the VCSEL electrical contacts to a plane that is substantially perpendicular to the surface of the laser emitting spot. This enables the optical link module to be mounted on a carrier such as a flexible PCB within the limited space in the catheter while the VCSEL is optically aligned and connected to a fiber that is running in parallel to the carrier and the catheter shaft.

The interconnection unit 30 comprises a contact layer 50 which defines these flexible interconnects. The contact layer has a first portion 51 for connection to the attachment surface of the integrated circuit laser. In Figure 3, this first portion is beneath the laser 22 (hence cannot be seen) and faces upwardly. A second portion 52 is for electrical and mechanical connection of the interconnection unit 30 to a carrier 16, namely the flexible PCB. The contact layer 50 is configurable with a 90 degree bend between the first and second portions, as shown. It is "configurable" in the sense that it may be manufactured in a planar state and then deformed into the desired end configuration.

A silicon receiving block 54 is provided for receiving the optical fiber 24. The first portion 51 of the contact layer 50 is over an end face of the receiving block (the top face in Figure 3), and the receiving block is for aligning the end of the optical fiber 24 with the emitting region of the attachment surface of the integrated circuit laser 22.

The contact layer 50 is also formed over a silicon support block 56. The second portion 52 of the contact layer is connected over the support block 56. The support block is then connected or attached to the side face of the silicon receiving block 54.

The electrical tracks of the contact layer 50 are metal lines which have been realized by deposition of a continuous metal layer, followed by patterning this layer into tracks by means of lithographic techniques (applying a photoresist layer, masked exposure, development, and finally selective removal by etching). Thus, the contact layer is formed by a photolithographic process, over the silicon, and before the patterning of the silicon layer to form the blocks.

A region 58 between the receiving block and the support block is a bending zone. This region 58 is a locally thinner part of the silicon body beneath the contact layer, whereas the receiving block and support block are locally thicker regions of the silicon body.

In the folded configuration shown, the bottom face of the support block (the face opposite the second portion 52) butts against a side face of the receiving block 54, and indeed this sets the 90 degree angle between the first and second portions 51, 52. In alternative embodiments the contact layer in folded configuration may be such that the first and second portions 51, 52 form an angle anywhere between 70 to 110 degrees.

In alternative configuration the receiving block 54 may have slanted face adjacent the end face on which the first portion 51 of the contact layer is attached, and the second portion 52 of the contact layer 50 is attached to the slanted lateral face of the receiving block by the support block 56. The slant may be positive or negative. Particular advantage is when the slant is positive, and the angle formed between the first and second portions of the contact layer is between 90-110 degrees, as the configuration may be used for a better electrical contact alignment and mechanical connection of the second portion 52 to the carrier 16, e.g. the flexible PCB. The wedge-like longitudinal cross section of the receiving block in the interconnection unit 30 can serve for positioning the assembly in the interventional medical device 10 along the longitudinal axis during manufacturing, as the carrier 16 located complementarily slanted in the interventional medical device prevents further advancing in longitudinal direction of the interconnection unit and provides the desired necessary mechanical and electrical contact of the second portion of the contact layer to the carrier. In further alternative embodiment the material of the receiving block 54 may be different from Si.

Figure 4 shows a second perspective view of the optical link module and shows that the receiving block 54 comprises a through hole 60 for receiving the optical fiber 24. The through hole extends to the end face at which the laser 22 is mounted.

Note that instead of a through hole with a circular opening, other opening shapes are possible. Also, instead of an enclosed shape, there could be a slot or channel so that the optical fiber can be inserted into the receiving block from the side. This may be give a simpler or just alternative manufacturing process.

In this example, the receiving block 54 is a cuboid shaped element, which has a planar dimension of 240 µm x 240 µm and a thickness of 400 µm. The intermediate contact pads for VCSEL connection are fabricated on the 240 µm x 240 µm end face and the through-silicon hole 60 for the optical fiber goes through the two opposing 240 µm x 240 µm surfaces. The laser, in particular VCSEL, with a dimension of 235 µm x 235 µm x 150 µm, is flip-chipped on intermediate contact pads of the first portion 51 of the contact layer, with its laser emitting spot aligned to the optical fiber via the through silicon hole 60. Both the through silicon hole 60 and the intermediate contact pads are designed and fabricated in accordance with the VCSEL layout. Therefore, a multimode optical fiber that is inserted from the bottom side into the through silicon hole 60 will be automatically aligned to the laser emitting spot of the VCSEL from the other side.

Standard flip-chip assembly techniques guarantee an alignment accuracy within 10-20 µm, which is sufficient to align a multimode fiber. The through silicon hole 60 also serves as a mechanical support for the multimode optical fiber. With a small dot of (epoxy) adhesive, the fiber can be fixated in the through silicon hole 60 and a reliable connection can be guaranteed.

The support block 56 has a planar dimension of 240 µm x 380 µm and a thickness of 40 µm. The rerouted electrical contacts for the PCB connection are fabricated on the second portion of the contact layer 52, which is over a 240 µm x 380 µm top surface.

Flexible interconnects along the contact layer connect these rerouted electrical contacts to the intermediate contact pads on which the VCSEL is mounted.

Initially, the first and second portions 51, 52 are in the same plane. Thus, the contact layer is manufactured in a planar configuration. The flexible interconnect allows folding of the thinner support block 56, by means of the bending zone 58, by 90 degrees onto the side of the cuboid receiving block 54. The two blocks are fixed in this position with an adhesive.

Hence, the folding separates the VCSEL laser emitting spot and the rerouted electrical contacts into perpendicular planes. By connecting the rerouted electrical contacts to the carrier 16, in particular a flexible PCB, the optical link module acts as a stand-alone device, which fits inside the catheter together with other components, as shown in Figures 1 and 2.

The interconnection unit 30 may be formed using so-called Flex-to-Rigid (F2R) technology. The F2R technology is an interconnect manufacturing platform that has been developed to integrate complex electronic sensing and imaging functionality on the tip of catheters and guidewires. It is based on the same microfabrication technologies that are used for the fabrication of cMUT transducers. It allows for the fabrication of arbitrary shaped silicon islands, of arbitrary size and thickness that contain (cMUT) ultrasound transducers, sensors, ASICs and/or passive components.

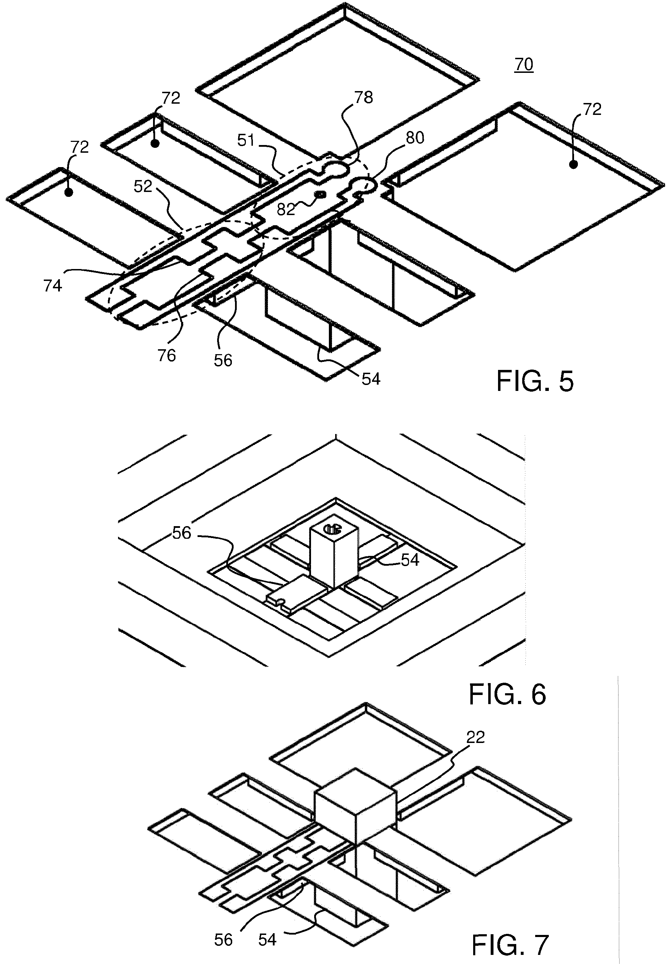

Figure 5 shows a silicon substrate 70 having through openings 72 and also different thickness of silicon on the underside. One thicker region (400 µm thick) defines the receiving block 54 and another thicker region (40 µm thick) defines the support block 56.

The bending zone is defined between the receiving block 54 and the support block 56, for example with thickness 5 µm to 6 µm.

The contact layer 50 is planar during manufacture, and the first and second portions 51, 52 can be identified in Figure 5.

The rerouted electrical contacts 74 and 76 can be seen in Figure 5 as well as the contact pads 78 and 80 for electrical connection to the integrated circuit laser. There is also an opening 82 for alignment with the emitting region of the laser.

Figure 6 shows the underside of the arrangement of Figure 5.

During assembly, the structure shown in Figures 5 and 6 is initially formed.

As shown in Figure 7, the laser 22 is then mounted over the first portion 51 of the contact layer. The laser is mounted upside down on the first portion. This step is for example performed while the interconnection unit is still suspended in the support wafer although it could be performed after separation.

Figure 7 shows the score lines for cutting support tabs and thereby separating the optical link module.

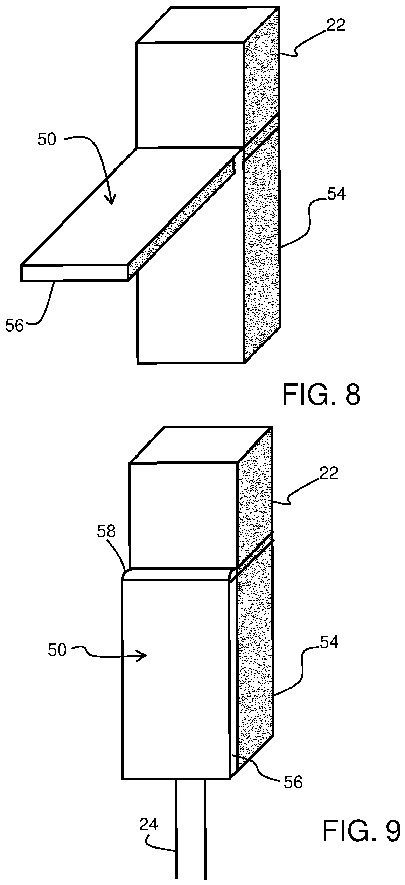

Figure 8 shows the separated optical link module, with the contact layer 50 still planar.

Figure 9 shows the folding of the contact layer 50 around the bending zone 58 to form the final configuration, such as the second portion of the contact layer together with the support block are over a side face of the receiving block, and the insertion of the optical fiber 24. The receiving block and support block are attached to each other, e.g. glued together and the optical fiber is attached, e.g. glued in place. This gluing may either take place before or after mounting the interconnection unit including pre-assembled VCSEL sideways on the flex-PCB 16.

The example above shows an interconnection unit for one optical fiber and one laser, hence an optical link module for one laser-fiber coupling.

The optical link module construction can instead be adapted to connect multiple optical fibers in a row. This is interesting in a broad range of optical fiber coupling devices. Conventionally, this is achieved by adding discrete optical components to redirect the light from out-of-plane to in-plane and vice versa.

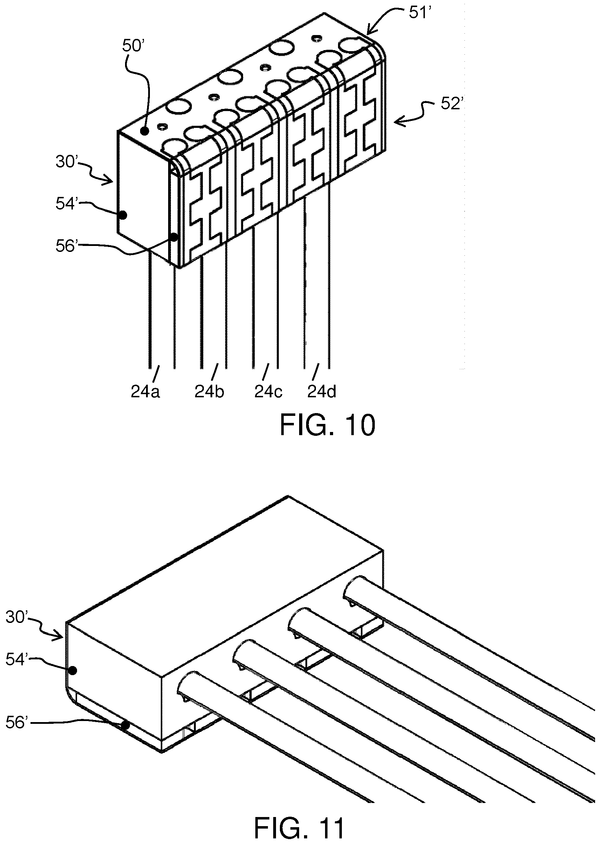

Figures 10 and 11 show different views of an interconnection unit 30' for coupling the output of a plurality of lasers to respective optical fibers 24a-24d. The first portion 51' of the contact layer 50' is then for connection to the attachment surface of each of a plurality of integrated circuit lasers. The second portion 52' is however for electrical and mechanical connection of the interconnection unit to a single carrier. The receiving block 54' thus comprises a plurality of through holes each for receiving a respective optical fiber.

This provides a simplification to the process of connecting multiple lasers, resulting in significant savings in component costs as well as in assembly costs. Moreover, it results in a much lower profile optical connection.

The design is compatible with standard VCSEL arrays, which are commercially available e.g. in 4x1 or 12x1 array elements with a standard pitch of 250 µm.

The examples above all relate to the delivery of optical signals into the optical fiber, for example from a sensor at the distal end of a catheter. Furthermore, the examples above all relates to lasers with a single surface having the electrical contacts as well as the laser output spot.

The invention may also be applied to a laser in which the electrical contacts are on one surface and the laser spot is on an opposite surface.

The invention may also be applied to an optical unit which is for receiving light from the optical fiber rather than delivering light to the optical fiber. The optical unit for example comprises a photodiode, and it may also have the electrical contacts on one surface and the light receiving region is on an opposite surface. Indeed, photodiodes generally have electrical contacts at their backside, i.e. the opposite side of the light receiving side.

Thus, a single fiber connection approach or a multi-fiber connection approach may also be used on the receiving photodiode side, i.e. at the opposite end of the optical fibers where the optical signals need to be detected.

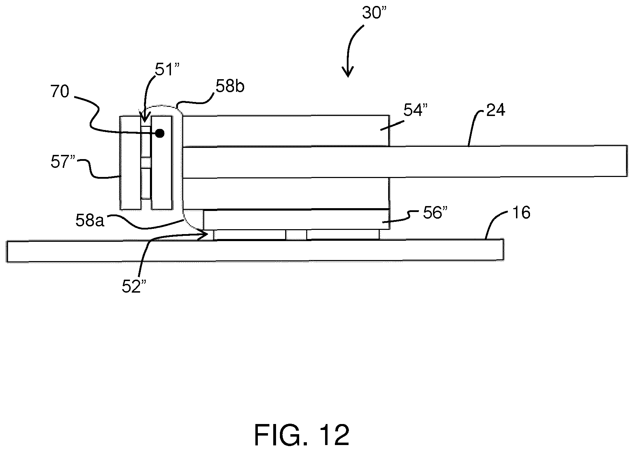

Figure 12 shows a different design of the interconnection unit suitable for optical units with the light receiving or emitting surface opposite the attachment surface.

The interconnection unit 30" again comprises a contact layer with a first portion 51" for connection to the attachment surface of the optical unit, which is for example a photodiode 70.

The attachment surface is opposite the light receiving surface and hence faces away from the end of the optical fiber 24, whereas the attachment surfaces faces the optical fiber end. The first portion 51" is thus over the top of the photodiode 70.

The second portion 52" is for electrical and mechanical connection of the interconnection unit 30 to the carrier 16, such as a PCB.

The contact layer 50 is in this case configurable with three 90 degree bends between the first and second portions, as shown. The first bend 58a leads to a region over the end of the receiving block 54". This may have an opening for the laser light received.

A 180 degree bend 58b (i.e. second and third 90 degree bends) leads to the top side of the photodiode 70. The end result is a 90 degree angle between the portions 51" and 52", but in this case the first portion 51" faces towards the end of the optical fiber instead of facing away from the optical fiber.

The first portion 51" of the contact layer 50 is still over an end face of the receiving block but it is also over the photodiode 70.

The silicon support block 56" again has the second portion 52" of the contact layer.

There is a second support block 57" in this design which carries the first portion 51" of the contact layer.

As described above, one application of interest is for catheters equipped with ultrasound imaging capability. Inter-cardiac ultrasound (ICE) catheters are well known. They typically incorporate piezoelectric or single crystal ultrasound elements at the catheter tip, for performing localized imaging. Other designs make use of capacitive micro-machined ultrasound transducers (CMUTs). CMUT devices are becoming increasingly popular because they can offer excellent bandwidth and acoustic impedance characteristics, which makes them the preferable over e.g. piezoelectric transducers. Vibration of the CMUT membrane can be triggered by applying pressure (for example using ultrasound) or can be induced electrically.

CMUT devices enable an array of transducers to be formed, for example so that electronic beam-forming in 2D or 3D becomes possible. CMUT array devices require local integrated circuitry (in particular ASICs) to enable operation of the CMUT elements. For example, high voltage pulses of a pulser circuit and a high voltage DC bias are generated by a probe circuit, which is typically an application specific integrated circuit (ASIC) which is located within the ultrasound probe, i.e. at the probe location.

The ASIC facilitates both transmission and reception modes of the device. In reception mode, changes in the membrane position cause changes in electrical capacitance, which can be registered electronically. In transmission mode, applying an electrical signal causes vibration of the membrane. Besides the ASICs, several capacitors are also needed to enable a stable power supply to the ASIC and CMUT. These circuit components are shown in Figure 1.

There are two main options for the CMUT arrangement and ASIC circuitry. A first option is to provide ASIC functionality below the CMUT drums, resulting in a monolithically integrated approach. The CMUT is then processed using thin film technology on top of an ASIC wafer. In this case, part or all of the ASIC functionality is provided on the rigid tip part with the CMUT drums. Additional functionality, such as passive components or further integrated circuits are then provided on the flexible carrier set back from the catheter tip.

A second option is to have separate ASIC functionality and CMUT drums. In this case, the ASIC functionality may be provided on a flexible carrier set back from the catheter tip together with the passive components.

All of these options are possible using the interconnect of the invention.

While the invention is of interest for ultrasound imaging, it may be used for converting any electrical element signals from electrical to optical using a laser, for coupling to an optical fiber for signal transmission.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An interconnection unit (30) for coupling an optical unit to an optical fiber (24), comprising:
a contact layer (50) having:
a first portion for connection to an attachment surface of the optical unit; and
a second portion (52) for electrical and mechanical connection of the interconnection unit to a carrier (16), wherein the contact layer (50) is configurable with an angle between the first and second portions (51,52); and
a receiving block (54) for receiving the optical fiber (24), wherein the first portion (51) of the contact layer (50) is over an end face of the receiving block (54), and the receiving block is for aligning the end of the optical fiber (24) with an optical emitting region or optical receiving region of the optical unit (22).

2. The interconnection unit as claimed in claim 1, wherein the contact layer (50) comprises flexible electrical connections (74, 76, 78, 80) formed over a substrate.

3. The interconnection unit as claimed in claim 1 or 2, wherein the receiving block (54) comprises a through hole (60) or channel for receiving the optical fiber, wherein the through hole or channel extends to the end face.

4. The interconnection unit as claimed in any one of claims 1 to 3, wherein the first portion (51) of the contact layer comprises contact pads (78, 80) for electrical connection to the attachment surface of the optical unit.

5. The interconnection unit as claimed in any one of claims 1 to 4, further comprising a support block (56), wherein the second portion (52) of the contact layer is over the support block.

6. The interconnection unit as claimed in claim 5, wherein the receiving block and the supporting block comprise silicon substrate portions.

7. The interconnection unit as claimed in claim 6, comprising a locally thinner substrate portion (58) between the receiving block and the supporting block, wherein the locally thinner substrate portion forms a bend zone for forming the angle between the first and second portions of the contact layer.

8. The interconnect unit of any of claims 1 to 7, wherein the angle is in the interval of 70-110 degrees.

9. The interconnection unit as claimed in any one of claims 1 to 8, for coupling the output of a plurality of optical units to respective optical fibers,
wherein the first portion (51') is for connection to the attachment surface of each of a plurality of optical units,
wherein the second portion (52') is for electrical and mechanical connection of the interconnection unit to a single carrier,
and wherein the receiving block (54') comprises a plurality of through holes each for receiving a respective optical fiber.

10. The interconnection unit as claimed in any one of claims 1 to 9, wherein:
the optical unit comprises an integrated circuit laser (22), wherein the attachment surface includes the optical emitting region and electrical connection regions; or
the optical unit comprises an integrated circuit laser (22), wherein the attachment surface includes electrical connection regions and is opposite a surface which includes the optical emitting region; or
the optical unit comprises a photodiode, wherein the attachment surface includes electrical connection regions and is opposite a surface which includes the optical receiving region.

11. A sensing interventional medical system comprising:
an interventional medical device (10) having a proximal end and a distal end;
a sensor element (12) positioned along the interventional medical device or at the distal end, the sensor element generating an electrical sensing signal;
an integrated circuit laser (22) for converting the electrical sensing signal to an optical sensing signal, wherein the integrated circuit laser comprises an attachment surface which comprises an emitting region and electrical connection regions;
an optical fiber (24) for relaying the optical sensing signal along the interventional medical device (10), the optical fiber having an end facing the emitting region;
the interconnection unit (30) as claimed in any one of claims 1 to 10 between the integrated circuit laser (22) and the optical fiber (24), wherein the contact layer (50) of the interconnection unit forms an angle between the first and second portions (51, 52) such that the second portion (52) is aligned to a side face of the receiving block in the direction of an axis of the interventional medical device;
a carrier (16) connected to the second portion (52) of the contact layer (50); and
circuitry (20) for controlling the integrated circuit laser, mounted on the carrier (16).

12. The system as claimed in claim 11, wherein:
the integrated circuit laser (22) is a vertical cavity surface emitting laser, VCSEL; and/or
the optical fiber (24) is a multimode optical fiber.

13. The system as claimed in claim 11 or 12, comprising a plurality of sensor elements and a respective plurality of optical fibers (24a-24d), wherein the first portion of the contact layer (50') is connected to the attachment surface of each of a plurality of integrated circuit lasers, wherein the second portion (52') of the contact layer is connected to a single carrier, and wherein the receiving block (54') comprises a plurality of through holes each for receiving the respective optical fiber.

14. A method of forming a coupling arrangement for coupling an optical unit to an optical fiber (24), the method comprising:
forming an interconnection unit comprising:
a planar contact layer (50) having a first portion (51) for connection to an attachment surface of the optical unit and a second portion (52) for electrical and mechanical connection to a carrier;
a receiving block (54) configured for receiving the optical fiber and aligning the end of the optical fiber with the optical receiving region or the optical emitting region of the optical unit, wherein the first portion of the planar contact layer is over an end face of the receiving block;
the second portion of the contact layer comprising a support block (56);
mounting the attachment surface of the optical unit over the first portion (51) of the planar contact layer (50);
forming an angle between the first and second portions (51, 52) of the contact layer; and
attaching the support block to a side face of the receiving block.

15. The method as claimed in claim 14, comprising:
mounting the coupling arrangement over the end of an optical fiber (24);
coupling the second portion of the contact layer to a carrier (16) which carries circuitry (20) for controlling the optical unit.
